# EUROPÄISCHE PATENTSCHRIFT

(11) **EP 1 242 354 B1**
(45) Veröffentlichungstag und Bekanntmachung des Hinweises auf die Patenterteilung: **20.07.2005**
(21) Anmeldenummer: 00991277.5
(22) Anmeldetag: 29.12.2000
(51) Int. Cl.: C07C 51/265, C07C 63/16, B01J 19/00

(54) **VERFAHREN UND VORRICHTUNG ZUR KÜHLUNG EINES HEISSEN REAKTIONSGASES, Z.B. BEI DER HERSTELLUNG VON PHTHALSÄUREANHYDRID**
METHOD AND DEVICE FOR COOLING A HOT REACTOR GAS, FOR EXAMPLE IN THE PRODUCTION OF PHTHALIC ANHYDRIDE
PROCEDE ET SYSTEME DE REFROIDISSEMENT D'UN GAZ REACTIONNEL A HAUTE TEMPERATURE, PAR EXEMPLE LORS DE LA PRODUCTION D'ANHYDRIDE PHTALIQUE

(30) Priorität: 30.12.1999 DE 19963869
(43) Veröffentlichungstag der Anmeldung: 25.09.2002
(60) Teilanmeldung: 05009314.5
(73) Patentinhaber: BASF AKTIENGESELLSCHAFT, 67056 Ludwigshafen (DE)
(72) Erfinder: WILKE, Alfred, 67166 Otterstadt (DE)
(74) Vertreter: Isenbruck, Günter, Dr.
(86) Internationale Anmeldenummer: PCT/EP2000/013329
(87) Internationale Veröffentlichungsnummer: WO 2001/049645

(56) Entgegenhaltungen:
- EP-A- 0 323 141
- GB-A- 1 422 516
- US-A- 3 466 300

## Beschreibung

Die Erfindung betrifft ein Verfahren zur Kühlung eines heißen Reaktionsgases auf eine vorherbestimmte Eingangstemperatur in einen Abscheider als Stufe bei der Herstellung von Phthalsäureanhydrid.

Ausgangspunkt der vorliegenden Erfindung bildet die Herstellung von Phthalsäureanhydrid (PSA) durch Oxidation von o-Xylol. Dabei werden heißes o-Xylol und heiße Luft in Gegenwart eines Katalysators umgesetzt. Das so gebildete PSA verläßt den Reaktor im Gemisch mit Luft als heißes Reaktionsgas und weist eine Temperatur von etwa 360°C auf. Bei PSA-Anlagen mit Nachreaktoren liegt diese Temperatur etwas niedriger. In einem nachgeordneten Abscheider erfolgt dann die Trennung in Roh-PSA und Luft. Vor Eintritt in den oder die Abscheider ist es erforderlich, das heiße Reaktionsgas auf eine vorherbestimmte gewünschte Temperatur, in der Regel etwa 160 bis 175°C, abzukühlen.

Üblicherweise wird hierfür ein komplizierter, mindestens zweistufiger Gaskühler mit mindestens zwei Dampftrommeln und entsprechenden Kühlmittelversorgungen eingesetzt. Dadurch wird das der eigentlichen Reaktion nachgeschaltete Aufbereitungsverfahren des Reaktionsgases aufwendig und kostenintensiv.

Es ist auch schon die Kombination von einem Haupt- mit einem nachgeschalteten Nachreaktor mit oder ohne darin enthaltener Kühlung beschrieben worden; beispielhaft sind die DE-A 197 42 821 oder die DE-A 198 07 018.

In der US 3,466,300 wird ein mehrstufiges (z.B. 14 Stufen) Verfahren zur Herstellung von PSA aus o-Xylol beschrieben, wobei das in die erste Stufe eingeführte Gemisch aus o-Xylol und Luft dadurch vorerhitzt wird, dass es zum Kühlen des die vier ersten Kontaktstufen verlassenden Reaktionsgases eingesetzt wird. Dabei werden Temperatursteigerungen in den ersten Stufen von 50 bis 60°C angestrebt, in den weiteren Stufen von 5 bis 15°C.

Aus der GB 1,422,516 ist ebenfalls ein Verfahren zur Herstellung von PSA aus o-Xylol und Luft bekannt, bei dem bestimmte Bedingungen (Salzbadtemperatur) einzuhalten sind. Es wird zwar auch von der Ausnutzung von entstehender Wärmeenergie (z.B. aus Wämneaustauschern, Nachreaktoren o.ä.) gesprochen, aber nicht ausschließlich gezielt zur Erhitzung der oder eines der Vorprodukte.

Der Erfindung liegt daher die Aufgabe zugrunde, ein optimiertes Verfahren zur Kühlung eines heißen Reaktionsgases bereitzustellen, das einen einfacheren und kostengünstigeren Aufbau einer Reaktionsanlage mit geringeren Wartungskosten ermöglicht, und zu einer qualitativ guten und von der Ausbeute her möglichst quantitativen Umsetzung der Edukte führt.

Gelöst wird diese Aufgabe durch ein Verfahren zur Herstellung von Phthalsäureanhydrid (PSA) durch Oxidation von o-Xylol in Gegenwart eines Katalysators, gegebenenfalls in 2 Stufen in einem Haupt- und Nachreaktor, bei dem das heiße Reaktionsgas aus dem Hauptreaktor enthaltend PSA und Luft dadurch auf eine vorherbestimmte Eingangstemperatur in einen Abscheider gekühlt wird, daß dem Abscheider ein Wärmetauscher vorgeschaltet wird, den ein Kühlmedium durchströmt, und das heiße Reaktionsgas zur Wechselwirkung mit dem Kühlmedium durch den Wärmetauscher geleitet wird, wobei die Eingangstemperatur des Reaktionsgases in den Wärmetauscher zwischen 250 und 400°C und seine Ausgangstemperatur aus dem Wärmetauscher zwischen 130 und 180°C liegt, die Eingangstemperatur des Kühlmediums in den Wärmetauscher liegt unterhalb von 100°C und seine Ausgangstemperatur aus dem Wärmetauscher zwischen 300 und 350°C, und das durch die Wechselwirkung mit dem Reaktionsgas erwärmte Kühlmedium wiederum als einer der Reaktanden für die chemische Umsetzung in den Reaktor geführt wird.

Der Wärmetauscher ersetzt den Gaskühler, wodurch die gesamte Reaktionsanlage einen vereinfachten Aufbau erhält und mit geringerem Kostenaufwand zu warten ist.

In einer bevorzugten Ausführungsform des erfindungsgemäßen Verfahrens liegt bei PSA-Anlagen ohne Nachreaktor die Eingangstemperatur des Reaktionsgases zwischen 350 und 380°C bzw. bei PSA-Anlagen mit Nachreaktor bei 280 bis 320°C. Die Ausgangstemperatur liegt vorzugsweise bei etwa 160°C. Der Wärmetauscher ist aber auch in der Lage, Reaktionsgase oder sonstige Gase mit anderen vorherbestimmten Eingangstemperaturen auf die genannte Ausgangstemperatur des Wärmetauschers oder wahlweise eine andere Ausgangstemperatur zu kühlen.

Besonders vorteilhaft ist, daß die Kühlung des Reaktionsgases auf die vorherbestimmte Eingangstemperatur in den Abscheider nach dem erfindungsgemäßen Verfahren in dem Wärmetauscher einstufig erfolgen kann.

Gemäß einer bevorzugten Ausführungsform des erfindungsgemäßen Verfahrens wird ein gasförmiges Kühlmedium und besonders bevorzugt wird Luft verwendet.

Die Ausgangstemperatur des Kühlmediums liegt vorzugsweise bei etwa 330°C. Die Temperatur des aus dem Wärmetauscher wieder austretenden Kühlmediums liegt nämlich gemäß dem erfindungsgemäßen Verfahren um etwa 150°C höher als dies nach dem im Stand der Technik bekannten Verfahren üblich ist. Dadurch hat es eine Temperatur, die ein direktes Einführen des Kühlmediums in den Reaktor erlaubt. Dabei wird das Kühlmittel so ausgewählt, daß es gleichzeitig einen der Reaktanden der chemischen Umsetzung darstellt.

In einer anderen Ausführungsform wird ein Teilstrom des Kühlmediums über einen Bypass um den Wärmetauscher geführt und mit dem aus den Wärmetauscher austretenden erhitzten Kühlmedium wieder vermischt. Dadurch läßt sich gezielt eine beliebige Temperatur des bevorzugt als Einsatzstoff verwendeten Kühlmediums einstellen. Vorzugsweise geschieht dies durch eine temperaturgeführte Bypassmengenregelung.

Als ein Reaktand wird heißes o-Xylol in Gegenwart eines Katalysators und heißer Luft als weiterem Reaktanden umgesetzt. Das durch die Umsetzung entstehende heiße Reaktionsgas besteht im wesentlichen aus PSA und Luft. Da Luft gleichzeitig das Kühlmedium für das heiße Reaktionsgas ist und - wie weiter oben geschildert - mit einer erheblich höheren Temperatur aus dem Wärmetauscher austritt als dies im Stand der Technik möglich ist, kann es direkt in den Reaktor geführt werden. Ein weiterer Vorteil ist, daß in die, die heiße Luft enthaltende Leitung das zunächst eine Temperatur von etwa 30°C aufweisende o-Xylol geführt und gleichzeitig ohne weiteren apparativen und energetischen Aufwand miterwärmt wird. Dennoch resultiert eine gegenüber dem Stand der Technik um etwa 100°C höhere Eintrittstemperatur in den einzigen oder den Haupt-Reaktor, was zu einer besseren Auslastung des Katalysators und einer geringeren Aufheizzone im Reaktor führt.

Demgegenüber war es bisher im Stand der Technik erforderlich, die Luft über einen Vorerwärmer umständlich und energieintensiv vorzuheizen. In diese vorerwärmte Luft wurde das in einem separaten, mit Dampf beheizten Wärmetauscher vorgeheizte flüssige o-Xylol eingespeist. Das Gemisch aus Luft und o-Xylol erreichte aber nicht die nach dem erfindungsgemäßen Verfahren erzielte Eintrittstemperatur in den Reaktor. Außerdem hat sich gezeigt, daß im Reaktor bei der erfindungsgemäßen Verfahrensweise mehr Hochdruckdampf erzeugt werden kann.

Im folgenden soll die Erfindung anhand von in der Zeichnung dargestellten Ausführungsbeispielen und -formen näher erläutert werden.

Es zeigen
- Fig. 1: ein Fließschema des im Stand der Technik bekannten Verfahrens zur Herstellung von PSA
- Fig. 2: ein Fließschema gemäß einer ersten Ausfühnmgsform des erfindungsgemäßen Verfahrens der PSA-Herstellung und der dazu geeigneten Vorrichtung
- Fig.3: ein Fließschema gemäß einer zweiten Ausführungsform des erfindungsgemäßen Verfahrens und der dazu geeigneten Vorrichtung
- Fig.4: ein Fließschema gemäß einer dritten Ausführungsform der Erfindung inkl. Bypassleitung um den Wärmetauscher mit Temperaturregelung
- Fig. 5 a,b: ein Fließchema gemäß einer vierten Ausführunsgform der Erfindung (zwei Plattentauscher inkl. Bypass mit Nachreaktor)
- Fig. 6 a-c: Ausführungsformen der Wärmetauscher mit Nachreaktor(2-er Kombinationen)
- Fig. 7: eine Ausführungsform ohne ersten Wärmetauscher

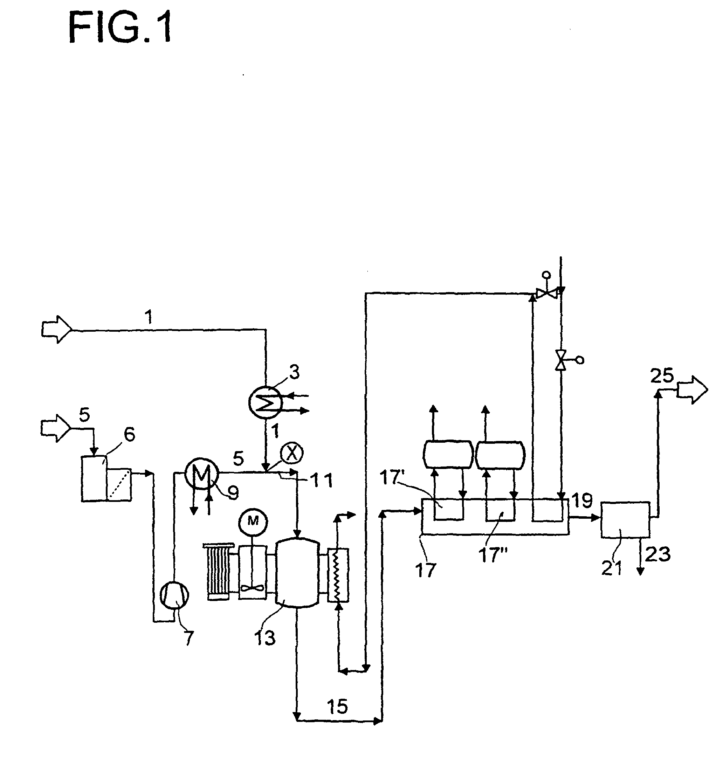

Zunächst wird in Fig. 1 eine Reaktoranlage dargestellt und erläutert, wie sie im Stand der Technik zur Herstellung von PSA bekannt ist. PSA kann bekanntlich durch Oxidation von o-Xylol in Gegenwart eines Katalysators hergestellt werden. Das eingesetzte o-Xylol weist zu Beginn eine Temperatur von etwa 30°C auf und wird über Leitung 1 einem Vorwärmer 3 zugeführt den es mit einer Temperatur von in etwa 135 bis 180°C verläßt. Anschließend wird es in Leitung 5 eingespeist. Über diese Leitung 5 wird Luft zugeführt, die zu Beginn ebenfalls eine Temperatur von etwa 30°C aufweist. Die Luft in Leitung 5 passiert zunächst ein Gebläse 7, wird dabei auf etwa 80 bis 90°C erwärmt und gelangt dann in einen Vorwärmer 9, den sie auf etwa 150 bis 200°C erwärmt wieder verläßt. Zu diesem Zeitpunkt wird das eine Temperatur von etwa 135 bis 180°C aufweisende o-Xylol aus Leitung 3 in Leitung 5 eingespeist. Das Luft/o-Xylol-Gemisch in Leitung 5 passiert eine Mantelleitungsheizung 11, die dazu dient, die Temperatur des Luft/o-Xylol-Gemisches bei etwa 160 bis 175°C zu halten und erreicht so den Reaktor 13 für die chemische Umsetzung.

Der Reaktor 13 stellt einen Rohrreaktor dar, in dessen Röhren sich der für diese Oxidationsreaktion erforderliche Katalysator, z.B. Cs dotiertes Vanadium/Titan-Oxid auf einem Träger wie Steatit, befindet.

Nach erfolgter chemischer Umsetzung verläßt das Reaktionsgas mit einer Temperatur von etwa 350. bis 375°C den Reaktor 13 über Leitung 15, es besteht im wesentlichen aus PSA und Luft; Nebenbestandteile sind z.B. Phthalid und Maleinsäureanhydrid. Von dort gelangt es zu einem zweistufigen Gaskühler 17, der zwei Dampftrommeln 17', 17" aufweist und wird auf eine Temperatur von etwa 160 bis 175°C abgekühlt. Damit weist das Reaktionsgas die vorherbestimmte günstige Temperatur auf, um über Leitung 19 zu einem Abscheider 21 geführt zu werden. Aus dem Abscheider 21 wird über Leitung 23 Roh-PSA entnommen und über Leitung 25 das Abgas einer Verbrennung oder Wäsche zugeführt.

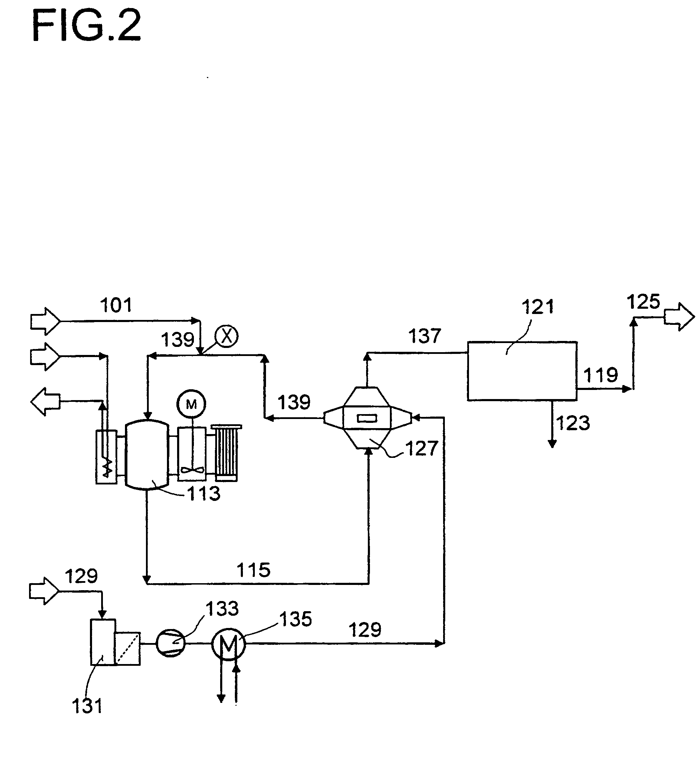

Demgegenüber ist in Fig. 2 das erfindungsgemäß geänderte Verfahren bzw. die dazu geeignete Vorrichtung dargestellt. Dabei werden solche Vorrichtungsmerkmale, die den in Fig. 1 bereits dargestellten entsprechen, mit den selben, jedoch um 100 erweiterten Bezugszahlen versehen.

Für die erfindungsgemäße Durchführung der chemischen Umsetzung wird anstelle des zweistufigen Gaskühlers ein einstufiger Wärmetauscher, hier ein Gas/Gas-Plattenwärmetauscher 127 verwendet. Dadurch kann die Reaktionsführung gleich mehrfach verändert werden und es ist daher vorteilhaft, die erfindungsgemäße Durchführung der Umsetzung ausgehend von dem Gas/Gas-Plattenwärmetauscher 127 zu beschreiben.

Dem Gas/Gas-Plattenwärmetauscher 127 wird aus dem Reaktor 113 strömendes Reaktionsgas, mit einer Temperatur von etwa 350 bis 375°C über Leitung 115 zugeführt. Gleichzeitig fließt über Leitung 129 ein Kühlmittel in Form von Luft mit einer Temperatur von etwa 90°C in den Plattenwärmetauscher 127 ein. Diese Kühlmittel-Luft weist zunächst eine Temperatur von etwa 30°C auf und wird über einen Luftfilter 131 geleitet, um anschließend mittels eines Gebläses 133 und eines Anfahrerhitzers 135 auf eine Temperatur von 90°C erwärmt zu werden. In dem Plattenwärmetauscher 127 findet nunmehr ein Wärmeaustausch zwischen dem heißen Reaktionsgas und der Kühlmittel-Luft statt. Das Reaktionsgas verläßt den Plattenwärmetauscher 127 anschließend über Leitung 137 mit einer Temperatur von etwa 160 bis 175°C. Damit weist das Reaktionsgas die vorherbestimmte günstige Temperatur auf, um dem Abscheider 121 zugeführt und anschließend auf die schon beschriebene Weise in Roh-PSA und Abgas getrennt zu werden. Durch den Wärmeaustausch zwischen dem heißen Reaktionsgas und der Kühlmittel-Luft wird letztere auf etwa 330°C erwärmt und kann jetzt über Leitung 139 dem Reaktor 113, der gleichartig zum bereits beschriebenen Stand der Technik ausgebildet ist, als einer der Reaktanden für die chemische Umsetzung zugeführt werden.

D. h. gegenüber dem bisher bekannten Verfahren wird die Kühlmittel-Luft um etwa 150° höher aufgeheizt, wodurch ihr erneuter Einsatz als ein Reaktand der chemischen Umsetzung ohne die bisher notwendige energieintensive Vorwärmung dieses Reaktanden über einen Vorwärmer (z.B. 9) möglich und sinnvoll ist.

Gleichzeitig entfällt aber auch die bisher notwendige Vorwärmung des o-Xylols über einen weiteren Vorwärmer (z.B. 3).

o-Xylol, das über Leitung 101 zugeführt wird, gelangt direkt in die die heiße Kühlmittel-Luft aufweisende Leitung 139 und es bildet sich das für die Umsetzung zu PSA benötigte o-Xylol/Luft-Gemisch. Durch die Zuführung des etwa eine Temperatur von 30°C aufweisenden o-Xylol kühlt sich das o-Xylol/Kühlmittel-Luft Gemisch zwar auf etwa 280°C ab. Diese Temperatur, mit der es dann in den Reakter 113 eintritt, liegt aber immer noch gut 100°C höher, als dies nach der bekannten Verfahrensweise möglich ist. Dadurch kann der Katalysator im Reaktor 113 besser ausgenutzt und mehr Hochdruckdampf erzeugt werden. Da die separate o-Yylol-Vorwärmung über einen Vonvärmer und z.B. die Mantelheizung 11 der in Fig. 1 zum Reaktor führenden Leitung 5 entfallen, wird der Anlagenaufbau einfacher und kostengünstiger, was ebenso für die Wartung der Anlage gilt.

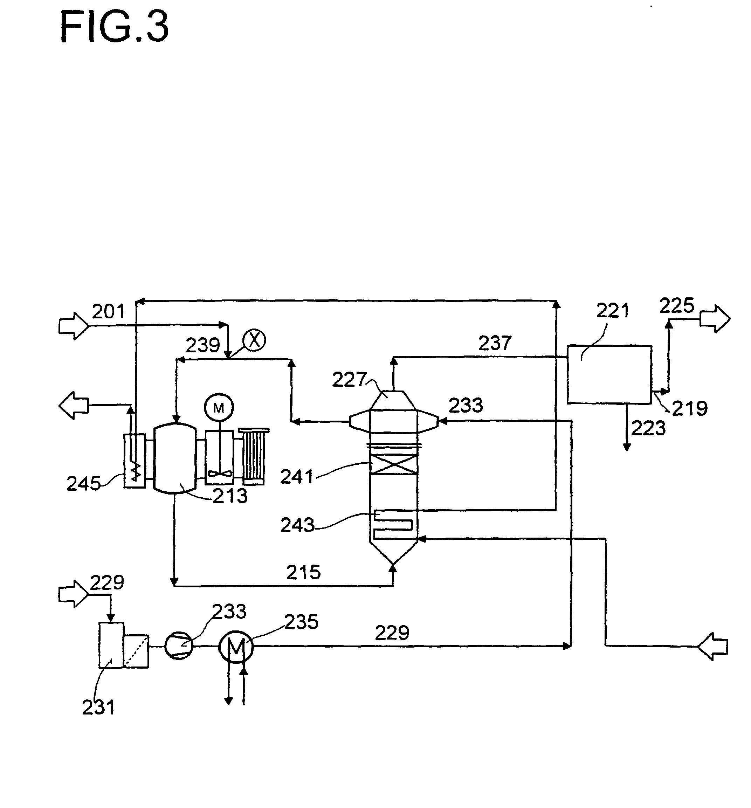

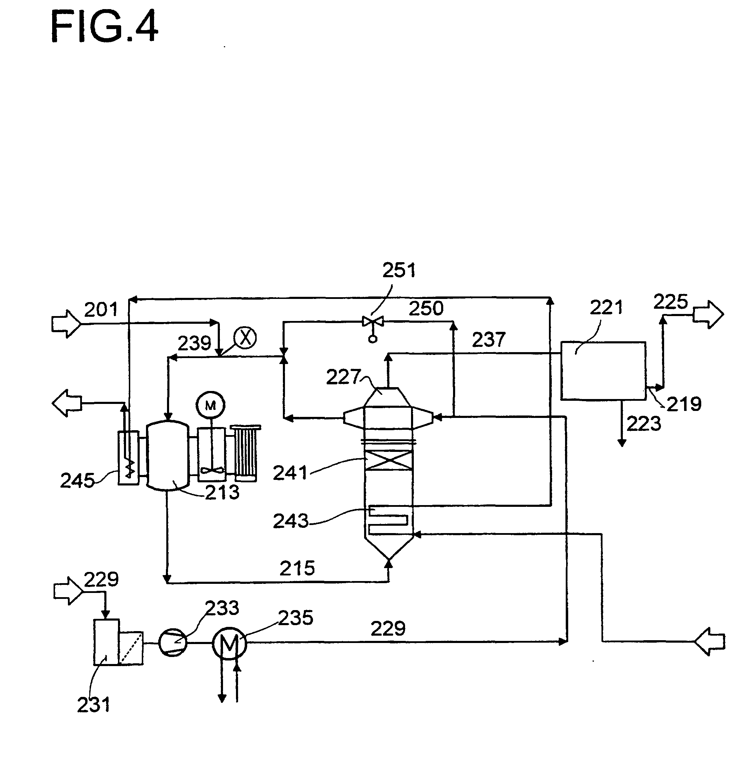

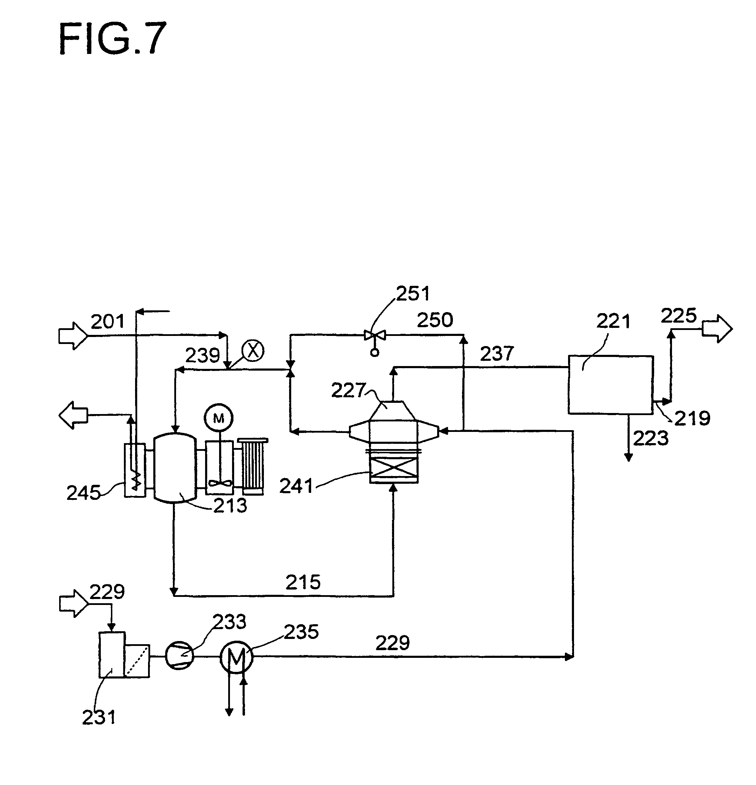

In Fig. 3 ist eine weitere Ausführungsform des erfindungsgemäßen Verfahrens und einer dazu geeigneten Vorrichtung dargestellt. Dabei werden die zu der Ausführungsform gemäß Fig. 2 gleichen Vorrichtungsmerkmale mit entsprechend gleichen, aber um hundert erweiterten Bezugszahlen versehen; diese Art der Bezugszahlen wird dann in den weiteren Figuren (d.h. Zahlen ab 200) beibehalten. Fig. 4 zeigt eine zu Fig. 3 analoge Anordnung mit einer Bypassleitung 250 einschl. einer Temperaturregelung 251, hingegen Fig. 7 eine sich von Fig. 4 unterscheidende Anordnung ohne den Zwischenkühler 243.

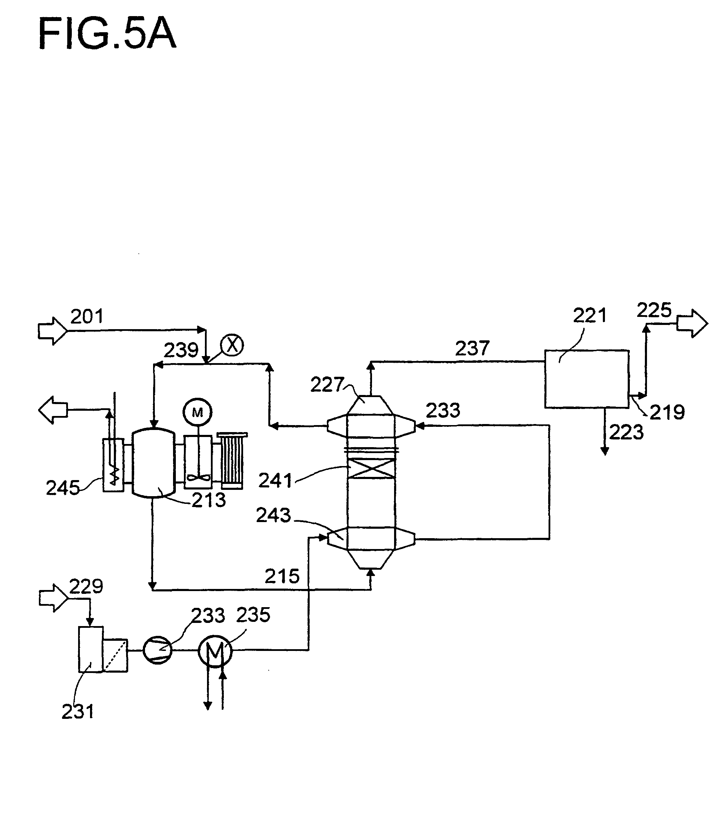

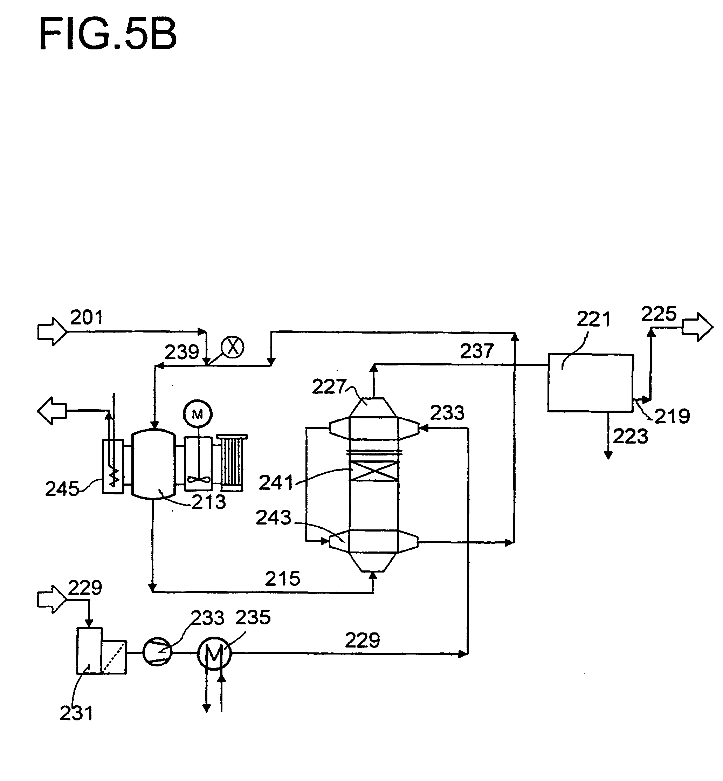

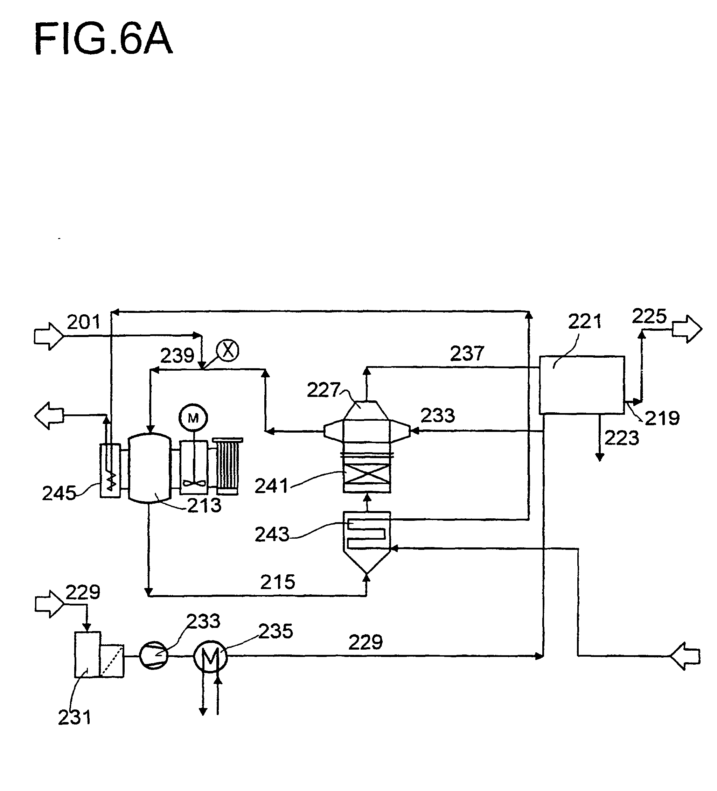

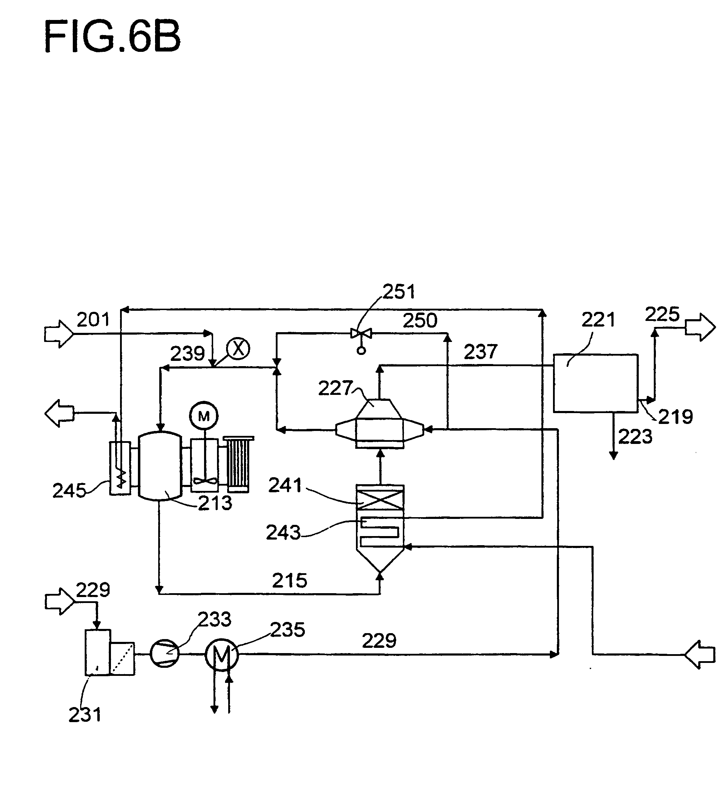

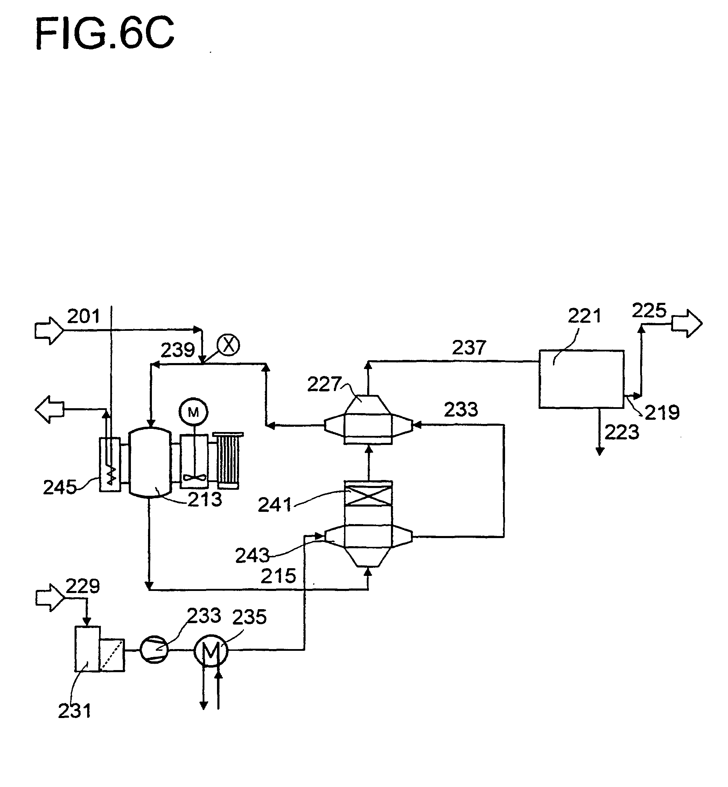

Die Ausführungsform gemäß Fig. 3 unterscheidet sich von der Ausführungsform gemäß Fig. 2 grundsätzlich nur dadurch, daß dem Reaktor 213 ein Nachreaktor 241 mit einem gleichen oder von dem im Hauptreaktor 213 verschiedenen Katalysator nachgeschaltet ist; ein geeigneter Katalysator für den Nachreaktor ist beispielsweise ein undotiertes Vanadium/Titan-Oxid auf einem Träger wie Siliciumcarbid. Da der Nachreaktor 241 eine niedrigere Eingangstemperatur aufweisen soll als das Reaktionsgas nach seinem Austritt aus dem eigentlichen Reaktor 213 aufweist, ist dem Nachreaktor 241 ein Zwischenkühler 243 vorgeschaltet. Der Zwischenkühler 243 kann dabei als Brauchwasservorerwärmer für einen Salzbadkühler 245, der dem Reaktor 213 zugeordnet ist, ausgeführt werden. Er kann aber genauso als Vorerwärmer für die Kühlmittel-Luft, z. B. vom Typ eines weiteren Gas/Gas-Plattenwärmetauschers oder eines anderen Wärmeaustauschertyps, ausgeführt werden. Dies ist in Fig. 5 a und b dargestellt, wobei dann z.B. die den einen Plattenwärmetauscher 243 (Zwischenkühler) verlassende Kühlmittel-Luft dem zweiten Plattenwännetauscher 227 als Kühlmittel-Luft (Fig. 5a) - oder auch in umgekehrter Folge (Fig. 5b) - zugeleitet werden kann. Dabei sind der Zwischenkühler 243, der Nachreaktor 241 und der Gas/Gas-Plattenwärmetauscher 227 in der Ausführungsform gemäß Fig. 3 in einem gemeinsamen Gehäuse angeordnet. Es hat sich jedoch gezeigt, daß es zur Optimierung der Reaktionserfordernisse auch sinnvoll sein kann, den Zwischenkühler 243, den Nachreaktor 241 und/oder den Gas/Gas-Plattenwärmetauscher 227 in voneinander getrennten Gehäusen anzuordnen. Dabei können alle drei Vorrichtungen in jeweils eigenen Gehäusen oder jeweils zwei der genannten Vorrichtungen wiederum zusammen in einem Gehäuse angeordnet sein. Diese Varianten sind in Fig. 6a (Trennung des Zwischenkühlers 243 von Nachreaktor 241 und Wärmetauscher 227), 6b (Trennung des Wärmetauschers 227 von Zwischenkühler 243 und Nachreaktor 241) und 6c (wie 6b ohne Bypass 250 aber 243 als Gas/Gas-Plattenwärmetauscher), jeweils mit einer Verbindung zwischen den Bauteilen dargestellt.

## Patentansprüche

1. Verfahren zur Herstellung von Phthalsäureanhydrid (PSA) durch Oxidation von o-Xylol in Gegenwart eines Katalysators, gegebenenfalls in 2 Stufen in einem Haupt- und Nachreaktor, bei dem das heiße Reaktionsgas aus dem Hauptreaktor enthaltend PSA und Luft auf eine vorherbestimmte Eingangstemperatur in einen Abscheider gekühlt wird, **dadurch gekennzeichnet, dass** dem Abscheider ein Wärmetauscher vorgeschaltet wird, den ein Kühlmedium durchströmt, und das heiße Reaktionsgas zur Wechselwirkung mit dem Kühlmedium durch den Wärmetauscher geleitet wird, wobei die Eingangstemperatur des Reaktionsgases in den Wärmetauscher zwischen 250 und 400°C und seine Ausgangstemperatur aus dem Wärmetauscher zwischen 130 und 180°C liegt, die Eingangstemperatur des Kühlmediums in den Wärmetauscher unterhalb von 100°C und seine Ausgangstemperatur aus dem Wärmetauscher zwischen 300 und 350°C liegt, und das durch die Wechselwirkung mit dem Reaktionsgas erwärmte Kühlmedium als einer der Reaktanden für die chemische Umsetzung in den Reaktor geführt wird.

2. Verfahren nach Anspruch 1, **dadurch gekennzeichnet, daß** die Kühlung des Reaktionsgases einstufig erfolgt.

3. Verfahren nach Anspruch 1, **dadurch gekennzeichnet, daß** ein gasförmiges Kühlmedium und zwar Luft verwendet wird.

4. Verfahren nach einem der Ansprüche 1 bis 3, **dadurch gekennzeichnet, dass** die Eingangstemperatur des Reaktionsgases in den Wärmetauscher bei PSA-Anlagen ohne Nachreaktor zwischen 350 und 380°C bzw. bei PSA-Anlagen mit Nachreaktor zwischen 280 bis 320°C und seine Ausgangstemperatur bei etwa 160°C liegt.

5. Verfahren nach einem der Ansprüche 1 bis 4, **dadurch gekennzeichnet, dass** die Ausgangstemperatur des Kühlmediums bei etwa 330°C liegt.

## Claims

1. A process for the preparation of phthalic anhydride (PAA) by oxidation of o-xylene in the presence of a catalyst, if appropriate in 2 stages in a main reactor and downstream reactor, in which the hot reaction gas from the main reactor, comprising PAA and air, is cooled to a predetermined inlet temperature on introduction into a separator, wherein a heat exchanger through which a cooling medium flows is located upstream of the separator; and the hot reaction gas is passed through the heat exchanger for interaction with the cooling medium, the inlet temperature of the reaction gas being from 250 to 400°C and its outlet temperature on emerging from the heat exchanger being from 130 to 180°C, the inlet temperature of the cooling medium on introduction into the heat exchanger being below 100°C and its outlet temperature on emerging from the heat exchanger being from 300 to 350°C, and the cooling medium heated by the interaction with the reaction gas being fed into the reactor as one of the reactants for the chemical reaction.

2. A process as claimed in claim 1, wherein the cooling of the reaction gas is effected in one stage.

3. A process as claimed in claim 1, wherein a gaseous cooling medium, specifically air, is used.

4. A process as claimed in any of claims 1 to 3, wherein the inlet temperature of the reaction gas on introduction into the heat exchanger is from 350 to 380°C in the case of PAA plants without a downstream reactor and from 280 to 320°C in the case of PAA plants with a downstream reactor and its outlet temperature is about 160°C.

5. A process as claimed in any of claims 1 to 4, wherein the outlet temperature of the cooling medium is about 330°C.

## Revendications

1. Procédé de préparation d'anhydride phtalique (PSA) par oxydation d'o-xylène en présence d'un catalyseur, éventuellement en deux étapes dans un réacteur principal et un réacteur subséquent dans lequel le gaz réactionnel chaud issu du réacteur principal et contenant du PSA et de l'air à une température d'entrée prédéterminée est refroidi dans un séparateur, **caractérisé en ce qu'**en amont du séparateur est monté un échangeur de chaleur que traverse un milieu de refroidissement et **en ce que** le gaz réactionnel chaud est conduit au travers de l'échangeur de chaleur en vue d'une interaction avec le milieu de refroidissement, la température d'entrée du gaz réactionnel dans l'échangeur de chaleur étant comprise entre 250 et 400°C et sa température de sortie de l'échangeur entre 130 et 180°C, la température d'entrée du milieu de refroidissement dans l'échangeur de chaleur étant inférieure à 100°C et sa température de sortie de l'échangeur de chaleur entre 300 et 350°C, et **en ce que** le milieu de refroidissement chauffé par l'interaction avec le gaz réactionnel est conduit dans le réacteur comme un des réactifs pour la réaction chimique.

2. Procédé suivant la revendication 1, **caractérisé en ce que** le refroidissement du gaz réactionnel a lieu en une étape.

3. Procédé suivant la revendication 1, **caractérisé en ce qu'**un milieu de refroidissement gazeux, notamment de l'air, est utilisé.

4. Procédé suivant l'une des revendications 1 à 3, **caractérisé en ce que** la température d'entrée du gaz réactionnel dans l'échangeur de chaleur est, pour des installations de PSA sans réacteur subséquent, comprise entre 350 et 380°C et, respectivement, pour des installations de PSA munies d'un réacteur subséquent, entre 280 et 320°C, sa température de sortie étant d'environ 160°C.

5. Procédé suivant l'une des revendications 1 à 4, **caractérisé en ce que** la température de sortie du milieu de refroidissement est d'environ 330°C.
